Europäisches Patentamt

(19) European Patent Office

Office européen des brevets

(11) EP 0 832 604 A1

(12) EUROPEAN PATENT APPLICATION

(43) Date of publication:
01.04.1998 Bulletin 1998/14

(51) Int. Cl.$^6$: A61B 8/06, G01S 15/89

(21) Application number: 96402081.2

(22) Date of filing: 30.09.1996

(84) Designated Contracting States:
AT BE CH DE DK ES FI FR GB GR IE IT LI LU MC NL PT SE

(71) Applicant:
PHILIPS ELECTRONICS N.V.
5621 BA  Eindhoven (NL)

(72) Inventor: Bonnefous, Odile
75008 Paris (FR)

(74) Representative:
Landousy, Christian
Société Civile S.P.I.D.
156, Boulevard Haussmann
75008 Paris (FR)

(54) Method and device for measuring the elasticity of an artery by ultrasonic echography

(57)  The invention relates to a method for measuring locally the elasticity $\gamma$ of an artery under the effect of the blood pressure P by means of a determination of instantaneous blood flow rates ($Q_A(t)$, $Q_B(t)$), instantaneous radius variations ($\Delta r_A(t)$, $\Delta r_B(t)$) and artery mean radius $r_0$ for two neighbouring, parallel excitation lines A and B traversing said artery according to one of its meridian planes. The method comprising some computing steps and the implementation of the least squares method applied, over a whole cardiac cycle or for each part of said cycle, to a relation including $\gamma$, followed by a determination step of the time varying part of $\gamma$ by means of a parametric modelization of the stress-strain relations of arterial walls.

Application : clinical examination of arteries by ultrasonic echography.

FIG.1

## Description

The present invention relates to a method for measuring locally the elasticity $\gamma$ of an artery under the effect of the blood pressure P by means of an ultrasonic echograph suitable to determine the instantaneous blood flow rates ($Q_A(t)$, $Q_B(t)$), the instantaneous radius variations ($\Delta r_A(t)$, $\Delta r_B(t)$) and the mean arterialy radius $r_0$ for two neighbouring, parallel excitation lines A and B traversing said artery according to one of its meridian planes, said method comprising the following steps :

(A) determining, on the basis of one of the two functions ($Q_A(t)$, $Q_B(t)$), the time mean value $Q_0$ and the derivative $\partial Q(t)/\partial t$ of the function $Q(t) = (Q_A(t) + Q_B(t)/2$ ;

(B) computing, on the basis of the instantaneous values ($\Delta r_A(t)$, $\Delta r_B(t)$), the dilation gradient $\delta r(t) = \Delta r_A(t) - \Delta r_B(t)$ ;

(C) determining the mean values $\gamma_0$, $R_0$, $R_0 Q_0$ of the elasticity $\gamma$, the hydrodynamic resistance R and the pressure gradient dp/dx respectively, said determination being carried out on the basis of the least squares method applied, over a whole cardiac cycle, to the following relation :

$$\gamma.(R.Q(t) + L.\frac{\partial Q}{\partial t} + \frac{dp}{dx}) = - \frac{\partial r(t)}{\partial x}$$

in which

$$L = \rho/\pi r_0^2 \, ,$$

$\partial r(t)/\partial x = \delta r(t)/e$ , $\rho$ is the voluminal mass of blood and e is the distance between the lines A and B.

The invention also relates to a corresponding device for measuring locally the elasticity $\gamma$ of an artery, allowing to carry out this method.

Diagnosis of vascular diseases and therapeutic choices are nowadays based on the analysis of the arterial lesions morphology and on the analysis of blood flows. This information is achieved with ultrasound imaging and Doppler examinations. These tools, although widely clinically validated, only get round the problem which can be formulated in the following manner : does the examined artery fulfil efficiently its function of pressure wave guide, conveying the kinetic power generated by the cardiac pump while adapting the wave form to the downstream arterial system ? To answer this question, the echographic tool must detect the beating artery and the flowing blood in order to reveal the mechanical working of the artery. The analysis of these observations, in the light of known blood flow and arterial strain models, must allow the characterization of the arterial mechanical properties. The a priori knowledge imbedded in these models is of course the cornerstone of this principle.

A measuring method as defined in the preamble of this text is for instance described in the document EP-0 603 967. This method already enables to deduce from some knowledge of the behaviour of a point of an artery the elasticity of said artery at this point.

The object of the invention is however to propose an improved measuring method, said improvement being based on a better knowledge of the mechanical behaviour of arterial walls, which is then formulated through a parametric stress-strain relation linking pressure and dilation variations.

At this end the invention relates to a method as described in the preamble, wherein said method comprises the following additional step :

(D) determining the time varying part of $\gamma$ by means of a parametric modelization of the stress-strain relations of arterial walls.

With the relation thus choosen, a more efficient measuring method is defined and carried out. For this implementation, the invention relates to a device for measuring locally the elasticity $\gamma$ of an artery under the effect of the blood pressure and comprising, in an ultrasonic echograph used in profilometer mode (M mode) and provided with transmitting and receiving means including a device for forming channels in the receiving mode, a first measuring sub-assembly for the determination of the instantaneous blood flow rates ($Q_A(t)$, $Q_B(t)$), the instantaneous radius variations ($\Delta r_A(t)$, $\Delta r_B(t)$) and the artery mean radius $r_0$ for two neighbouring, parallel excitation lines A and B, said lines traversing said artery according to one of its meridian planes and being situated at a given distance e from each other taken in the direction of the axis of the artery and comprised between some tenths of a millimeter and some millimeters, a second storing sub-assembly for storing at the output of said first sub-assembly digital samples of signals relating to the blood flows and signals relating to the walls of the artery, and a third computing sub-assembly which operate on said digital signal samples, wherein said third sub-assembly comprises computing means for determining the time varying part of $\gamma$ by using a parametric modelization of the stress-strain relations of arterial walls.

The invention will now be described in further detail with reference to the accompanying drawings, in which :

FIG.1 shows schematically the arterial set-up and the acquisition and control instrumentation allowing an implementation of the method according to the invention ;
FIG.2 shows measured pressure and dilation curves, and the pressure obtained from a conventional elastic model and from the viscoelastic model now proposed.

An ultrasonic procedure relying on the invention will now defined and its implementation explained. In vivo validation tests have been made, and an arterial set-up has been built, allowing in vitro measurements in physiological conditions as close as possible from living conditions and their confrontation with analytical models.

After a recall on blood flow models and on the measurements methods which allow to determine the parameters governing this flow, elastic and viscoelastic models of arterial walls are presented. Then the experimental validation of these models is described.

Blood flow models and measurements are first discussed. The Navier-Stokes equation describing flow velocities and pressure in a tube by linking pressure gradient and blood flow parameters can be expressed by the following equation (1) :

$$- \frac{dp}{dx} = RQ + L \frac{\partial Q}{\partial t} \tag{1}$$

where dp/dx, the pressure gradient along the x axis of the tube, generates a flow Q. The blood viscosity creates a constant hydrodynamic resistance R to the flow, and the blood inertia is responsible for the $L\partial Q/\partial t$ term, L being equal to $\rho/\pi r^2_0$ ($r_0$ = mean arterial radius, $\rho$ = voluminal mass of blood).

The variations of the artery size with time allow local blood storage followed by restitution of blood volume. The storage takes place during systole (cardiac ejection) while the volume flow is restituted during diastole (no cardiac outflow). This regulation of flow describes the basic arterial function. It closely depends on the arterial ability to be dilated with the pressure wave propagation. The distensibility, or elasticity, is expressed through the definition of the arterial compliance, C :

$$C = \frac{\partial S}{\partial p} \tag{2}$$

which allows to eliminate the pressure gradient term in the Navier-Stokes equation (1) and to write :

$$- \frac{1}{C} \frac{\partial S}{\partial x} = RQ + L \frac{\partial Q}{\partial t} \tag{3}$$

leading to an expression linking flow (Q) and cross-section (S), independently of the pressure, and implying intrinsic mechanical variables of blood (R,L) and of arterial wall (C). It is the basis of the analysis method that has to be implemented. Non invasive ultrasonic measurements of the volume flow Q and arterial diameter r (which is related to the cross-section S) allow the estimation of R, L and C, through an appropriate numerical identification method between the two equation members [1]. These measurements are simultaneously carried out on two longitudinal close positions, in order to get $\partial S/\partial x$, Q and $\partial Q/\partial t$. The measurement methods are time domain correlation techniques applied to ultrasonic signals, which track the echoes with time and allow the estimation of the displacements of the corresponding biological structures [2]. The flow curve Q(t) is derived by integration of the velocity profiles measured along the acoustic beam axis. The arterial diameters r(x,t) are determined by locally measuring displacements of arterial front and posterior walls and combining them to finally obtain the arterial dilation [3].

The arterial compliance is a non linear function of the pressure. Besides, this non linearity gives its genuine flow regulation ability to the artery. The inversion of equation (3) is tremendously complicated since it is necessary to introduce this non linarity in the left term. It is now convenient to discuss the model considered for the arterial compliance, i.e. the elastic and viscoelastic models of arterial walls.

The viscoelastic behaviour of arteries is due to their complex structure. The compliance is dependent on the local arterial pressure at each instant. Many studies done to express diameter/pressure relations are described in the literature. Most of the proposed models are derived from experimental data. Others result from elasticity theory.

The model initially used derives from the one of Languewouters and al [4]. They have measured in vitro the arterial cross-section as a function of the pressure, this latter being statically set, and proposed a parametrical law giving the arterial section as a function of pressure. This law is here transformed without changing its meaning, to get the arterial pressure P and the compliance C as functions of the arterial radius r :

$$P(r) = K \tan(\alpha + \beta r), \tag{4}$$

$$C(r) = 2\pi r_0 \cdot \frac{\partial r}{\partial p} = 2\pi r_0 \cos^2(\alpha + \beta r)/(k\beta). \tag{5}$$

K, $\alpha$, $\beta$ are the model parameters ($r_0$ is the mean arterial radius, as already seen). The identification method mentioned in the above paragraph consists in tuning the parameters K, $\alpha$, $\beta$, in order to verify at best identity (3), by minimizing the resulting quadratic error.

The Languewouters law, obtained in static pressure conditions, corresponds to the hypothesis of pure elastic behaviour of the arterial wall. It is validated in vivo by Y. Tardy et al [5] in the particular cases of humeral and brachial arteries, which are relatively little distendable. In vivo experiments carried out on the carotid artery, using this pure elastic model and undertaking the measurements of volume flow and arterial dilations previously described, have never enabled to determine R, L, C with a satisfactory confidence level. This contradictory results re-open the question of the initial pure elastic hypothesis and lead finally to the introduction of a viscous component in the parietal behaviour, that neither Languewouters in vitro experiments nor in vivo Tardy ones could evidence.

In the case of viscoelastic medium, the stress (pressure P) depends on both strain (r) and strain speed ($\partial r/\partial t$). The graphic representation P(r) shows an hysteresis characteristic of viscous phenomenons. For modeling this hysteresis, it is chosen to parametrize separately the two phases of the cardiac cycle (systole, diastole), while keeping the general relations proposed by Languewouters. Thus, two sets of parameters K, $\alpha$, $\beta$, corresponding respectively to the arterial expansion and its return to the initial state, allow to completely describe the viscoelastic behaviour of the arterial wall.

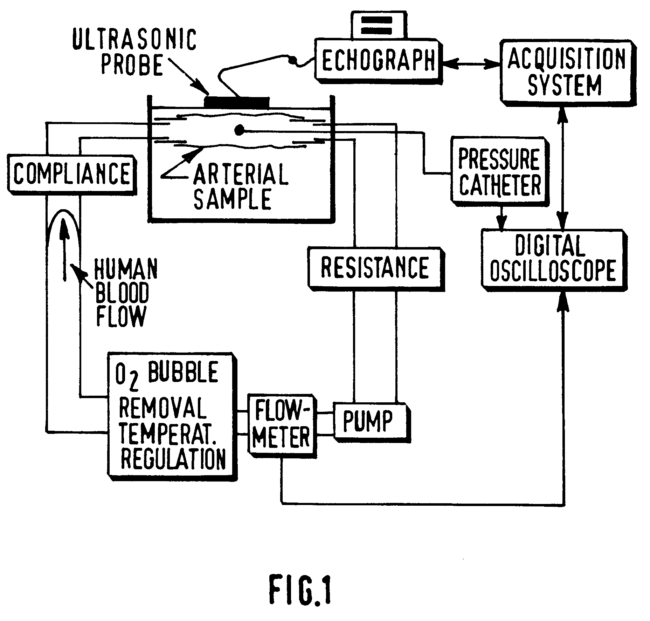

The validation of the viscoelastic model described above required confrontation with experimental data, by simultaneous measurements of arterial dilation and arterial pressure. It needed the building of an arterial set-up reproducing as well as possible the physiological conditions, and the implementation (around it) of the necessary control and measurement instrumentation. Figure 1 shows a schematic representation of this arterial set-up and of the related acquisition and control instrumentation.

An arterial sample freshly taken from a cadaver is placed in a temperature regulated conservation bath. It is perfused with total human blood supplied by a blood transfusion center. The hematocrit is controlled during the experiment to verify the hemolysis degree and the blood viscosity. Heparin is used to prevent any coagulation of the system. The hemodynamic set-up is made up of an extracorporal peristaltic pump interfaced with a frequency generator allowing pulsed flow generation, and of a dedicated conduction equipment ensuring a low hemolysis. A compliance and a resistance allow to adjust the flow and pressure curves. A heat exchanger and an extracorporal circulation module are place in parallel to ensure a constant temperature, to oxygenate and eliminate bubbles.

The instrumentation allows on the one hand to control flow conditions, using an external flow-meter and manometer, and on the other hand to acquire data. A pressure catheter provides the intra luminal pressure, and an echograph allows the recording of acoustic signals. The pressure acquisition is performed using a digital oscilloscope, the ultrasound signals are stored in a dedicated acquisition system, and these two recordings are synchronized to avoid an artificial delay which would hide any viscous behaviour. The arterial diameter variations are derived by applying the ultrasound signal processing techniques previously described.

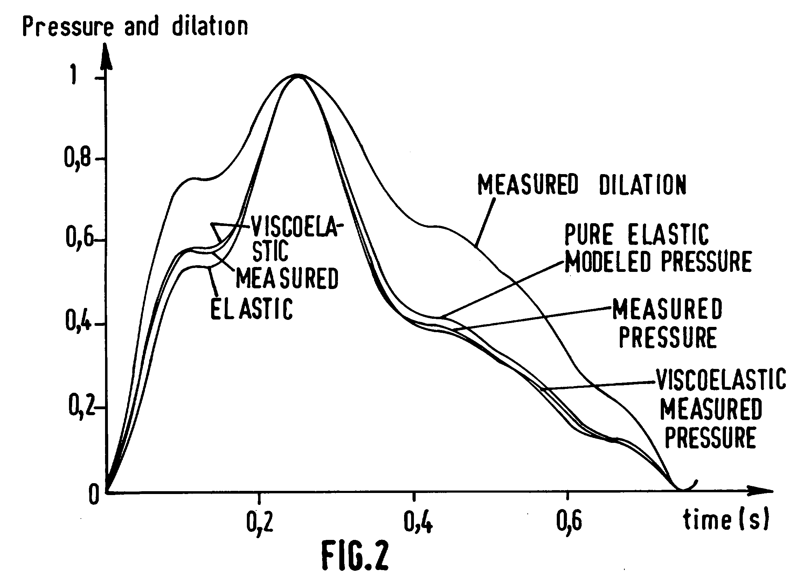

The confrontation of the measurements and of the viscous or viscoelastic models are performed by minimizing the quadratic error between the measured pressure and the modelled one, the coefficient set K, $\alpha$, $\beta$ being adapted to realize this minimization. Using data extracted from seven common carotid arteries, the two models are assessed through the residual quadratic error. In all the cases, this error is above 10 % for the pure elastic model and below 1 % for the viscoelastic one. Figure 2 illustrates the model validity, by showing the superposition of measured dilation, and pressure curves as functions of time.

From a more precise mathematical point of view, the general equation linking the pressure gradient and blood flow parameters, given by the relation (1), can also be expressed as follows :

$$\frac{dp}{dx} = (\frac{\partial p}{\partial x})r_0 + \frac{\partial p}{\partial r} \cdot \frac{\partial r}{\partial x} \tag{6}$$

The first term

$$(\partial p / \partial x)_{r_0}$$

is here considered as a constant term, responsible for the continuous component of the flow $RQ_0$. The time varying term of the pressure gradient :

$$\frac{dp(t)}{dx} = \frac{\partial p}{\partial r} \cdot \frac{\partial r}{\partial x} - \frac{\partial p}{\partial x}$$

is linked to the elasticity of the artery and is related to the compliance relation (7), equivalent to the relation (5) :

$$C = \frac{\partial S}{\partial p} = 2\pi r_0 \cdot \frac{\partial r}{\partial p} = 2\pi r_0 \gamma \tag{7}$$

The initial equation can be rewritten in the following way (with $q = Q - Q_0$) :

$$R_q + L \frac{dq}{dt} = -\frac{1}{\gamma} \frac{\partial r}{\partial x} = -\frac{dp}{\partial x} \tag{8a}$$

$$RQ_0 = (\frac{\partial p}{\partial x}) r_0 \tag{8b}$$

A resolution using the mean square method then consists in minimizing the quadratic error :

$$E^2 = \| R_q + L \frac{dq}{dt} + \frac{dp}{dx} \|^2$$

The constants R and L are easily extracted from the equations $\partial E^2 / \partial R = 0$ and $\partial E^2 / \partial L = 0$, which gives :

$$R = \frac{\langle q \cdot \frac{dq}{dt} \rangle \langle \frac{dp}{dx} \cdot \frac{dq}{dt} \rangle - \langle \frac{dq}{\partial t}^2 \rangle \langle q \cdot \frac{dp}{dx} \rangle}{\langle \frac{dq}{dt}^2 \rangle \langle q^2 \rangle - \langle q \cdot \frac{dq}{dt} \rangle^2} \tag{9a}$$

$$L = \frac{\langle q \cdot \frac{dq}{dt} \rangle \langle \frac{dp}{dx} \cdot q \rangle - \langle q^2 \rangle \langle \frac{dp}{dx} \cdot \frac{dq}{dt} \rangle}{\langle \frac{dq}{dt}^2 \rangle \langle q^2 \rangle - \langle q \cdot \frac{dq}{dt} \rangle^2} \tag{9b}$$

It can be noted that in the case of a cyclic phenomenon, when the summation involved by the integration ($\langle \rangle$ = mean value of the concerned function over a cardiac cycle) is done during this cycle, one has :

$$\langle q \cdot \frac{dq}{dt} \rangle = 0$$

which simplifies the previous expressions (9a) and (9b) and gives :

$$R = -\frac{\langle q \cdot \frac{dq}{dx} \rangle}{\langle q^2 \rangle} \tag{10a}$$

$$L = - \frac{\langle \frac{dp}{dx} \cdot \frac{dq}{dt} \rangle}{\langle \frac{dq}{dt}^2 \rangle} \qquad (10b)$$

The resulting quadratic error becomes :

$$E^2 = R^2 \langle q^2 \rangle + L^2 \langle \frac{dq}{dt}^2 \rangle + \langle \frac{dp}{dx}^2 \rangle + 2RL. \langle q. \frac{dq}{dt} \rangle + 2R. \langle \frac{dp}{dx}.q \rangle + 2L. \langle \frac{dp}{dx} \frac{dq}{dt} \rangle \qquad (11a)$$

and, in the case of a cyclic behaviour :

$$E^2 = \langle \frac{dp}{dx}^2 \rangle - \frac{\langle q. \frac{dp}{dx} \rangle^2}{\langle q^2 \rangle} - \frac{\langle \frac{dq}{dt} \cdot \frac{dp}{dx} \rangle}{\langle \frac{dq}{dt}^2 \rangle} \qquad (11b)$$

The final resolution of the equation (8a) consists in introducing an elasticity law in the expression of :

$$\frac{dp}{dx} = \frac{1}{\gamma} \cdot \frac{\partial r}{\partial x} \ ,$$

$\gamma$ being the compliance of the artery. This law is taken into account in computing the quadratic error, and the parameters inducing a minimum are considered as the solution of the problem. This compliance law is the basis of the resolution of the problem according to the invention. The time varying pressure gradient dp/dx is then modelized according to two different hypotheses :

- either the artery is purely elastic,
- or it is viscoelastic.

Considering the following model :

$$P_{\alpha,\beta}(r) = k.[\text{Arctg}(\alpha + \beta r) - \text{Arctg } \alpha] \qquad (12a)$$

$$(\frac{\partial p}{\partial x})_{\alpha,\beta} = \frac{dp}{dr} \cdot \frac{\partial r}{\partial x} = \frac{1}{k\beta.\cos^2(\alpha+\beta r)} \cdot \frac{\partial r}{\partial x} \qquad (12b)$$

and considering now the associated normalized quadratic error :

$$E^2_{n(\alpha,\beta)} = \frac{E^2_{\alpha,\beta}}{\langle (\frac{dp}{dx})_{\alpha,\beta} \rangle} \qquad (13)$$

where the formula (12b) is used to compute

$$E^2_{n(\alpha,\beta)} \ ,$$

the best solution is found for $(\alpha_S, \beta_S)$ minimizing this expression (13). It may be noted that the variation range of $\alpha$ and $\beta$ is the following :

- for $\alpha$ : $-\pi/2 \leq \alpha \leq \pi/2$

- for $\beta$ : $0 \leq \beta \leq ((\pi/2)-\alpha)/r_{max}$

With the first hypothesis of a purely elastic model, $\alpha$ and $\beta$ are considered as constant during the cardiac cycle : dilation and contraction of the artery are performed with the same stress-strain law. The quadratic error is then :

$$E^2_{n_{(\alpha,\beta)}} = 1 - \frac{\langle q.(\frac{dp}{dx})_{\alpha,\beta} \rangle^2}{\langle q^2 \rangle \langle (\frac{dp}{dx})^2_{\alpha,\beta} \rangle} - \frac{\langle \frac{dq}{dt}.(\frac{dp}{dx})_{\alpha,\beta} \rangle^2}{\langle \frac{dq}{dt}^2 \rangle \langle (\frac{dp}{dx})^2_{\alpha,\beta} \rangle} \qquad (14)$$

Now $\alpha_S$ and $\beta_S$ are determined, and k is computed thanks to the expression (10b) where L is already known :

$$k = -\frac{1}{L}.\frac{\langle \frac{1}{\beta_S.\cos^2(\alpha_S+\beta_S r)}.\frac{\partial r}{\partial x}.\frac{dq}{dt} \rangle}{\langle \frac{dq}{dt}^2 \rangle} \qquad (15)$$

Finally :

$$P(r) = k.[Arctg(\alpha_S + \beta_S r) - Arctg(\alpha_S)] \qquad (16)$$

With the second hypothesis of a viscoelastic model, the dilation and contraction phases of the artery correspond to the same kind of model but with different $(\alpha, \beta)$ solutions. That allows to introduce an hysteresis phenomenon in the stress-strain relations. However the resolution becomes more sophisticated since the term $\langle q.dq/dt \rangle$ cannot be set to 0, the integration being made on only a part of the cardiac cycle. For each part of the cardiac cycle, the following computation is then required :

(a) $R_{(\alpha,\beta)}$ and $L_{(\alpha,\beta)}$, with the formulas (9a) and (9b) ;
(b)

$$E^2_{n_{(\alpha,\beta)}} = E^2_{(\alpha,\beta)} / \langle (\frac{\partial p}{\partial x})^2_{\alpha,\beta} \rangle ,$$

this last computation of

$$E^2_{n_{(\alpha,\beta)}}$$

being derived from the expression (11a) by a division of all terms by

$$\langle (\frac{\partial p}{\partial x})^2_{\alpha,\beta} \rangle ,$$

with solutions $(\alpha_d, \beta_d)$ for the dilation and the solution $(\alpha_c, \beta_c)$ for the contraction, determined by minimizing

$$E^2_{n_{(\alpha,\beta)}} \quad ;$$

(c) a continuity condition is also necessary when the pressure and the arterial radius are maximum :

$$P(r_{max}) = k_d.[Arctg(\alpha_d + \beta_d \, r_{max}) - Arctg(\alpha_d)]$$
$$= k_c.[Arctg(\alpha_c + \beta_c \, r_{max}) - Arctg(\alpha_c)]$$

$$a = \frac{k_d}{k_c} = \frac{Arctg \, (\alpha_c+\beta_c r_{max}) - Arctg \, (\alpha_c)}{Arctg \, (\alpha_d+\beta_d r_{max}) - Arctg \, (\alpha_d)}$$

The final solution is therefore :

$$P_d(r) = k \times a.[Arctg(\alpha_d + \beta_d r) - Arctg(\alpha_d)]$$

$$P_c(r) = k.[Arctg(\alpha_c + \beta_c r) - Arctg(\alpha_c)]$$

with :

$$k = - T_d - T_c$$

and $T_d$, $T_c$ being given by :

$$T_d = \frac{1}{L}.\frac{\langle \dfrac{a}{\beta_d.\cos^2(\alpha_d+\beta_d r)} . \dfrac{\partial r}{\partial x} . \dfrac{dq}{dt} \rangle (dil)}{\langle \dfrac{dq}{dt}^2 \rangle (cycle)}$$

$$T_c = \frac{1}{L}.\frac{\langle \dfrac{1}{\beta_c.\cos^2(\alpha_c+\beta_c r)} . \dfrac{\partial r}{\partial x} . \dfrac{dq}{dt} \rangle (contr.)}{\langle \dfrac{dq}{dt}^2 \rangle (cycle)}$$

Thanks to this improved method, it is now possible to introduce an appropriate arterial strain law in vascular procedure. Assuming that this viscoelastic model is reliable, it is still necessary to define more precisely the convenient algorithm for the non invasive extraction of the arterial pressure. If successful, this new modality would lead to a genuine breakthrough of vascular echography, allowing the early detection of cardio-vascular diseases and improving the therapeutic follow-up.

[1]. O. BONNEFOUS. Device and method for measuring the elasticity of an artery by ultrasonic echography (US patent 5 411 028, priority of 22/12/92).

[2]. O. BONNEFOUS. Color Velocity Imaging : from an algorithm to a product. Revue Annuelle LEP 1990, (1991), 43-44.

[3.] O. BONNEFOUS. Vascular characterization with ultrasound. Revue Annuelle LEP 1992, (1993), 36-38.

[4]. G.J. LANGUEWOUTERS, K.H. WESSELING, W.J.A GOEDHARD. The static elastic properties of 45 human thoracic and 20 abdominal aortas in vitro and the parameters of a new model. J. Biomech, 17, (1984), 425-435.

[5]. T. ARDY, J.J. MEISTER, F. PERRET, H.R. BRUNNER, M. ARDITI. Non invasive estimate of the mechanical properties of peripheral arteries from ultrasonic and photoplethysmographic measurements. Clin.Phys.Physiol.Meas., 12, N°1, (1992), 39-54.

## Claims

1. A method for measuring locally the elasticity $\gamma$ of an artery under the effect of the blood pressure P by means of an ultrasonic echograph suitable to determine the instantaneous blood flow rates ($Q_A(t)$, $Q_B(t)$), the instantaneous

radius variations ($\Delta r_A(t)$, $\Delta r_B(t)$) and the mean arterial radius $r_0$ for two neighbouring, parallel excitation lines A and B traversing said artery according to one of its meridian planes, said method comprising the following steps :

(A) determining, on the basis of one of the two functions ($Q_A(t)$, $Q_B(t)$), the time mean value $Q_0$ and the derivative $\partial Q(t)/\partial t$ of the function $Q(t) = (Q_A(t) + Q_B(t)/2$ ;

(B) computing, on the basis of the instantaneous values ($\Delta r_A(t)$, $\Delta r_B(t)$), the dilation gradient $\delta r(t) = \Delta r_A(t) - \Delta r_B(t)$ ;

(C) determining the mean values $\gamma_0$, $R_0$, $R_0 Q_0$ of the elasticity $\gamma$, the hydrodynamic resistance R and the pressure gradient dp/dx respectively, said determination being carried out on the basis of the least squares method applied, over a whole cardiac cycle, to the following relation :

$$\gamma . (R.Q(t) + L.\frac{\partial Q}{\partial t} + \frac{dp}{dx}) = -\frac{\partial r(t)}{\partial x}$$

in which

$$L = \rho/\pi r_0^2,$$

$\partial r(t)/\partial x = \delta r(t)/e$ , $\rho$ is the voluminal mass of blood and e is the distance between the lines A and B, wherein said method comprises the following additional step :

(D) determining the time varying part of $\gamma$ by means of a parametric modelization of the stress-strain relations of arterial walls.

2.  A method as claimed in Claim 1, characterized in that said parametric stress-strain relation is defined by three parameters $\alpha$, $\beta$, k and according to relations of the following type :

$$\gamma_{\alpha,\beta,k} = k.\beta.\cos^2(\alpha + \beta r)$$

$$P_{\alpha,\beta,k} = k.(Arctg(\alpha + \beta r) - Arctg\alpha)$$

in which r is the artery radius variation under the effect of the blood pressure $P_{\alpha,\beta,k}$, and k is a proportionality coefficient, and in that the best determination $\gamma_{\alpha 0,\beta 0,k}$ of the compliance value $\gamma_{\alpha,\beta,k}$ measuring said elasticity is given by means of a computation, for each pair of parameters $\alpha$ and $\beta$, of a normalized quadratic error

$$E^2_{(\alpha,\beta)n}$$

defined by the relation :

$$E^2_{(\alpha,\beta)n} = \frac{\|R_q + L.\dfrac{dq}{dt} + \dfrac{1}{\gamma_{\alpha,\beta,k}}.\dfrac{dr}{dx}\|^2}{\|\dfrac{1}{\gamma_{\alpha,\beta,k}}.\dfrac{dr}{dx}\|^2}$$

in which $q = Q - Q_0$ , said computation being followed by the selection of the coefficients $\alpha_S$, $\beta_S$ that minimize said quadratic error.

3.  A device for measuring locally the elasticity $\gamma$ of an artery under the effect of the blood pressure and comprising, in an ultrasonic echograph used in profilometer mode (M mode) and provided with transmitting and receiving means including a device for forming channels in the receiving mode, a first measuring sub-assembly for the determination of the instantaneous blood flow rates ($Q_A(t)$, $Q_B(t)$), the instantaneous radius variations ($\Delta r_A(t)$, $\Delta r_B(t)$) and the mean arterial radius $r_0$ for two neighbouring, parallel excitation lines A and B, said lines traversing said artery according to one of its meridian planes and being situated at a given distance e from each other taken in the direction of the axis of the artery and comprised between some tenths of a millimeter and some millimeters, a second storing sub-assembly for storing at the output of said first sub-assembly digital samples of signals relating to the blood flows and signals relating to the walls of the artery, and a third computing sub-assembly which operate on said digital signal samples, wherein said third sub-assembly comprises computing means for determining the time

varying part of $\gamma$ by using a parametric modelization of the stress-strain relations of arterial walls.

FIG.1

Pressure and dilation

FIG.2

MEASURED DILATION

PURE ELASTIC MODELED PRESSURE

MEASURED PRESSURE

VISCOELASTIC MEASURED PRESSURE

VISCOELA-STIC MEASURED ELASTIC

time (s)

**European Patent Office**

## PARTIAL EUROPEAN SEARCH REPORT

which under Rule 45 of the European Patent Convention
shall be considered, for the purposes of subsequent
proceedings, as the European search report

Application Number

EP 96 40 2081

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | EP 0 603 967 A (PHILIPS ELECTRONICS N.V.) * the whole document * | 1-3 | A61B8/06 G01S15/89 |
| A | GB 2 156 985 A (TELTEC ELECTRONIC EQUIPMENT AB (SWEDEN)) * abstract * * page 6, line 51 - page 7, line 4; figures 1-6 * | 1-3 | |
| A | PROC. OF THE ANNUAL INTERNATIONAL CONF. OF THE IEEE ENGINEERING IN MEDICINE & BIOLOGY SOCIETY, vol. 15, no. 1, 28 - 31 October 1993, SAN DIEGO (US), pages 204-205, XP000436720 P.J. DHAWALE ET AL.: "In Vivo Estimation of Arteries with Intracoronary Ultrasound" * the whole document * | 1-3 | |
| A | US 5 099 852 A (J.J. MEISTER ET AL.) * the whole document * | 1-3 | TECHNICAL FIELDS SEARCHED (Int.Cl.6) A61B G01S |

### INCOMPLETE SEARCH

The Search Division considers that the present European patent application does not comply with
the provisions of the European Patent Convention to such an extent that it is not possible to carry
out a meaningful search into the state of the art on the basis of some of the claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 9 April 1997 | Hunt, B |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C07)

European Patent Office

EP 96 40 2081 - C -

## INCOMPLETE SEARCH

The Search Division considers that the present European patent application does not comply with the provisions of the European Patent Convention to such an extend that it is not possible to carry out a meaningfull search into state of the art on the basis of some of the claims.

Claims searched completely: 3
Claims searched incompletely: 1,2
Claims not searched:

Reason for the limitation of the search: Method for treatment of the human or animal body by surgery of therapy (see Art. 52(4) of the European Patent Convention).

EPO Form Supplementary Sheet C (1996)